# FASCICULE DE BREVET EUROPEEN

(11) **EP 4 284 254 B1**
(45) Date de publication et mention de la délivrance du brevet: **01.10.2025**
(21) Numéro de dépôt: 22706643.8
(22) Date de dépôt: 26.01.2022
(51) Int. Cl.: A61B 8/06, A61B 8/08, A61N 7/02

(54) **DISPOSITIF ET PROCÉDÉ DE CARACTÉRISATION DE L'ÉVOLUTION DU PROFIL DE VITESSE D'ÉCOULEMENT DE FLUIDE AU NIVEAU D'UNE ZONE DE TRAITEMENT PAR ÉMISSION D'ÉNERGIE**
VORRICHTUNG UND VERFAHREN ZUR CHARAKTERISIERUNG DER ENTWICKLUNG DES GESCHWINDIGKEITSPROFILS DES FLÜSSIGKEITSSTROMS AUF DER EBENE EINER BEHANDLUNGSZONE DURCH EMISSION VON ENERGIE
DEVICE AND METHOD FOR CHARACTERIZING THE EVOLUTION OF THE FLUID FLOW SPEED PROFILE AT THE LEVEL OF A TREATMENT ZONE BY EMISSION OF ENERGY

(30) Priorité: 26.01.2021 FR 2100694
(43) Date de publication de la demande: 06.12.2023
(73) Titulaire: EDAP TMS France, 69120 Vaulx-en-Velin (FR); Institut National de la Santé et de la Recherche Médicale (I.N.S.E.R.M.), 75013 Paris (FR); Université Claude Bernard Lyon I, 69100 Villeurbanne (FR); Centre Léon Bérard, 69008 Lyon (FR)
(72) Inventeur: CILLEROS, Celia G, 69100 VILLEURBANNE (FR); VINCENOT, Jérémy, 69100 VILLEURBANNE (FR); MELO DE LIMA, David, 01600 Saint Bernard (FR)
(74) Mandataire: Cabinet Beau de Loménie
(86) Numéro de dépôt international: PCT/FR2022/050139
(87) Numéro de publication internationale: WO 2022/162308

(56) Documents cités:
- US-A1- 2003 028 111
- US-A1- 2007 004 984
- US-A1- 2008 125 657

## Description

### Domaine technique

L'invention concerne le domaine technique des appareils ou des dispositifs comportant une sonde d'émission d'énergie, une sonde d'imagerie ultrasonore et un système de commande de la sonde d'émission et de la sonde d'imagerie ultrasonore. L'invention trouve une application particulièrement avantageuse dans le domaine de l'application d'ultrasons focalisés de haute intensité (HIFU) et de la caractérisation de la sténose qu'elle peut provoquer dans les vaisseaux sanguins.

### Technique antérieure

Il est connu que le traitement par application d'énergie, comme les ondes ultrasonores focalisées par exemple, permet de générer des destructions, par exemple par la chaleur, au niveau d'une zone focale avec des dimensions maitrisées entrainant des lésions irréversibles et détruisant les tissus biologiques visés. Une application particulière est alors le traitement des tumeurs.

Néanmoins, ce type de traitement n'est pas sans risque pour les vaisseaux, notamment sanguins, présents dans la zone de traitement. A titre d'exemple, l'échauffement ultrasonore pour détruire une tumeur péri-artérielle peut entraîner une occlusion du vaisseau sanguin en question. Ainsi, ces tumeurs ne peuvent pas être traitées par application d'énergie sans risque.

Il est connu de visualiser par imagerie les lésions provoquées par cette énergie pour évaluer l'avancement du traitement et déterminer l'opportunité de le poursuivre et/ou de l'adapter, comme cela est notamment décrit dans les documents US 2008/0125657, US 2007/004984 et US 2003/0028111. Une imagerie de la zone traitée est réalisée afin de visualiser les lésions provoquées par le traitement, mais ne permet pas de visualiser l'impact du traitement à la périphérie de la zone de traitement. De plus, lors du traitement, de nombreuses réactions interviennent au niveau de la zone de traitement (ébullition, cavitation, nécrose, changements importants de température...) . Ces facteurs impactent tous la qualité d'une acquisition échographique.

Afin de mettre en place un traitement plus sûr pour les patients, il est nécessaire de disposer d'un dispositif permettant non seulement l'émission d'énergie pour générer la lésion des tissus biologiques cibles, mais également permettant de caractériser le flux dans les vaisseaux périphériques à ces tissus biologiques, et ce de préférence en temps réel lors du traitement impliquant l'application d'énergie. Cela permettrait de limiter le risque d'une éventuelle occlusion de ces vaisseaux et de contrôler l'émission d'énergie afin de préserver ces vaisseaux.

Dans ce contexte, la Demanderesse a cherché à mettre au point un cycle de fonctionnement pendant lequel une énergie est successivement émise sur une zone de traitement, puis arrêtée pendant une période définie afin d'évaluer l'écoulement de fluide dans un vaisseau aux abords de la zone de traitement, et donc évaluer l'impact de l'application d'énergie sur ce vaisseau dans la zone de traitement et aux abords de celle-ci. Ainsi, l'objectif est une caractérisation suite à une application d'énergie, et en particulier entre des phases d'émission d'énergie, des modifications du flux au sein de vaisseaux aux abords de la zone de traitement provoquées lors de l'émission de l'énergie, ce qui permet d'évaluer l'impact de l'application d'énergie sur ce même vaisseau au niveau de la zone de traitement.

Différentes méthodes sont connues pour caractériser l'écoulement de fluide au sein de vaisseaux, et notamment de vaisseaux sanguins. Parmi celles-ci, la Demanderesse s'est plus particulièrement intéressée à l'imagerie ultrasonore, et en particulier l'imagerie Doppler.

L'imagerie ultrasonore a, en effet, déjà été utilisée pour caractériser les lésions, et les sténoses. L'imagerie Doppler permet, en particulier, de mesurer l'écoulement de fluide dans un vaisseau sanguin. L'imagerie ultrasonore présente l'avantage d'être rapide et fiable. Plusieurs types d'imagerie ultrasonore sont connus et couramment utilisés.

L'imagerie échographique ultrasonore conventionnelle (aussi appelée échographie en Mode B) est une imagerie en deux dimensions (2D) qui permet de visualiser les tissus biologiques dans la zone où est positionné le champ de visée de la sonde ultrasonore. Cette imagerie ne permet donc pas d'évaluer la vitesse d'écoulement de fluide dans un vaisseau, mais permet de visualiser les tissus biologiques dans une zone. Cette imagerie est donc utile pour visualiser et cibler les tissus biologiques cibles (comme une tumeur par exemple) ou le vaisseau à surveiller.

L'imagerie Doppler en une dimension (1D) renseigne sur la vitesse d'écoulement du fluide dans un vaisseau en un point ou segment où est localisée la zone de mesure. Cette imagerie ne permet donc pas de visualiser les tissus biologiques environnants, mais uniquement la courbe de signal Doppler indiquant l'évolution de la vitesse d'écoulement au cours du temps du fluide sur un segment déterminé.

L'imagerie Doppler couleur permet de visualiser le sens d'écoulement des flux dans une zone 2D ajustable au sein de l'image échographique. La couleur rouge ou bleu indique le sens de ce flux. Cette imagerie est donc utile pour visualiser les flux, notamment sanguins, dans une zone de traitement avant et après un traitement par émission d'énergie par exemple, ce qui peut permettre de mettre en évidence une sténose par exemple.

L'imagerie Doppler puissance est basée sur l'énergie du signal Doppler recueillie dans une zone 2D ajustable de l'image échographique. Cette technique est plus sensible dans la détection de flux et permet ainsi de visualiser des vaisseaux plus petits que l'imagerie Doppler couleur mais ne donne pas de renseignements sur la direction du flux.

Parmi les autres types d'imagerie Doppler connus, le mode TM (temps-mouvement) permet l'étude des mouvements des différentes structures, notamment cardiaques, les dimensions des cavités, notamment cardiaques, et l'épaisseur des parois.

La demande US 2010/0274133 décrit la détection d'une sténose d'un vaisseau sanguin par la mesure Doppler du flux turbulent dans ce vaisseau sanguin. La sonde Doppler est placée à distance de la zone de traitement, en aval de la sténose, perpendiculairement à l'écoulement sanguin dans le vaisseau, afin de détecter la turbulence du flux sanguin générée par la sténose. Le positionnement perpendiculaire au flux sanguin est important car il permet de mesurer uniquement l'écoulement turbulent, et de s'affranchir de l'écoulement laminaire le long du vaisseau sanguin. Il n'est alors plus possible de déterminer la vitesse d'écoulement du sang dans le vaisseau sanguin. De plus, le vaisseau sanguin n'est plus visualisé dans sa longueur, mais seulement selon une section. Les tissus biologiques, et notamment la tumeur que l'on cherche à détruire, ne peut plus être visualisée. Un traitement par émission d'énergie est alors difficilement envisageable en parallèle de cette détection de sténose.

### Exposé de l'invention

La Demanderesse a développé un dispositif permettant de caractériser l'évolution de l'écoulement du flux dans des vaisseaux à la périphérie de la zone de traitement, provoquée par l'émission d'énergie. Avantageusement, le dispositif selon l'invention permet non seulement de visualiser les modifications des tissus biologiques au sein de la zone de traitement, mais également la modification du profil de vitesse dans les vaisseaux aux abords de celle-ci, ces modifications étant provoquées par l'émission d'énergie. Pour cela, le dispositif pour la caractérisation d'une évolution du profil de vitesse d'écoulement de fluide à la périphérie d'une zone de traitement suite à l'émission d'énergie par une sonde d'énergie, grâce à une sonde d'imagerie ultrasonore dont la zone de mesure est placée en dehors de la zone de traitement, comprend un circuit de traitement du signal Doppler reçu, la sonde d'imagerie ultrasonore étant positionnée pour réaliser un plan d'image dans lequel l'écoulement de fluide est observé selon son axe longitudinal, ledit dispositif comportant un système de commande de la sonde d'émission d'énergie et de la sonde d'imagerie ultrasonore, configuré pour :
- commander en émission la sonde d'émission d'énergie selon des phases d'émission d'énergie successives entrecoupées par des phases d'arrêt de l'émission d'énergie, lesdites phases d'arrêt de l'émission d'énergie ayant une durée allant de 1,5 s à 15 s et de préférence de 1,5 s à 2,5 s,
- piloter en émission la sonde d'imagerie ultrasonore avant une phase d'émission d'énergie afin de mesurer un signal Doppler de référence et pendant les phases d'arrêt de l'émission d'énergie pour mesurer un signal Doppler reçu après chaque phase d'émission d'énergie),
- et permettre de fournir un suivi de l'évolution du profil de vitesse d'écoulement du fluide issu de l'imagerie ultrasonore.

Ce dispositif permet de réaliser une mesure de la vitesse d'écoulement du flux grâce à la sonde d'imagerie ultrasonore entre chaque phase d'émission d'énergie, et ainsi de déterminer l'évolution de ce flux dans le temps, et plus particulièrement dans les vaisseaux à proximité de la zone de traitement. La courbe du signal Doppler permet d'avoir une indication de l'impact de l'application d'énergie sur le vaisseau, ce qui permet d'évaluer l'état de santé du vaisseau, voire d'anticiper une occlusion de celui-ci.

Le dispositif selon l'invention peut également présenter l'une ou l'autre des caractéristiques suivantes, ou une quelconque combinaison de deux ou plusieurs de ces caractéristiques :
- la sonde d'imagerie ultrasonore donne accès à au moins un mode courbe Doppler, tel que le mode Doppler 1D ou le mode TM, pour mesurer une évolution du profil de vitesse d'écoulement de fluide au niveau de la zone de mesure, et au moins un mode d'imagerie en deux dimensions, tel que le mode B ou le mode Doppler couleur, pour visualiser des tissus biologiques de la zone de traitement,
- le système de commande pilote en émission la sonde d'émission d'énergie selon des phases d'émission d'énergie successives entrecoupées par des phases d'arrêt de l'émission d'énergie, selon un rapport cyclique compris entre 30% phase d'émission d'énergie /70% phase d'arrêt d'émission d'énergie et 90% phase d'émission d'énergie / 10% phase d'arrêt d'émission d'énergie
- le circuit de traitement du signal Doppler analyse l'évolution du profil de vitesse obtenu par effet Doppler,
- le circuit de traitement du signal Doppler comporte un système de comparaison de la courbe du signal Doppler reçue et la courbe du signal Doppler de référence après chaque phase d'émission d'énergie, pour déterminer l'évolution du profil de vitesse obtenu par l'imagerie ultrasonore,
- le système de comparaison du circuit de traitement du signal Doppler réalise la comparaison entre une moyenne de 3 à 5 courbes de signaux Doppler reçues et une moyenne de 3 à 5 courbes de signaux Doppler de référence,
- le circuit de traitement du signal Doppler détermine l'évolution du profil de vitesse grâce à la différence entre la(les) courbe(s) de signal(aux) Doppler reçue(s) et la(les) courbe(s) de signal(aux) Doppler de référence,
- le circuit de traitement du signal Doppler détermine l'évolution du profil de vitesse grâce à la dérivée de la différence entre la(les) courbe(s) de signal(aux) Doppler reçue(s) et la(les) courbe(s) de signal(aux) Doppler de référence,
- le circuit de traitement du signal Doppler comporte un système de comparaison à une valeur seuil de la (des) courbe(s) du signal Doppler reçue(s), de la différence entre la(les) courbe(s) de signal(aux) Doppler reçue(s) et la(les) courbe(s) de signal(aux) Doppler de référence, ou de la dérivée de la différence entre la(les) courbe(s) de signal(aux) Doppler reçue(s) et la(les) courbe(s) de signal(aux) Doppler de référence,
- le circuit traitement comporte un système de coupure arrêtant la sonde d'émission d'énergie lorsque la(les) courbe(s) de signal Doppler reçue(s), la valeur de la différence entre la(les) courbe(s) de signal(aux) Doppler reçue(s) et la(les) courbe(s) de signal(aux) Doppler de référence, ou la valeur de la dérivée de la différence entre la(les) courbe(s) de signal(aux) Doppler reçue(s) et la(les) courbe(s) de signal(aux) Doppler de référence atteint ladite valeur seuil,
- le dispositif comporte une base de données enregistrant les courbes de signal Doppler reçues, la différence des entre la(les) courbe(s) de signal(aux) Doppler reçu(s) et la(les) courbe(s) de signal(aux) Doppler de référence, et/ou la valeur de la dérivée de la différence entre la(les) courbe(s) de signal(aux) Doppler reçue(s) et la(les) courbe(s) de signal(aux) Doppler de référence afin de déterminer par lui-même la valeur seuil,
- la sonde d'émission d'énergie est une sonde d'émission d'ondes ultrasonores focalisées,
- la sonde d'émission d'énergie est une sonde d'émission d'électroporation, de radiofréquences ou une sonde d'émission de microondes.

L'invention concerne également un procédé de caractérisation d'une évolution du profil de vitesse d'écoulement de fluide à proximité d'une zone de traitement suite à une émission d'énergie à l'aide du dispositif selon l'invention, et comprenant les étapes suivantes :
a- positionnement de la zone de mesure de la sonde d'imagerie ultrasonore en dehors de la zone de traitement, à 5 à 60 mm de la zone de traitement, et longitudinalement par rapport à l'axe d'écoulement du fluide,
b- pilotage en émission la sonde d'imagerie ultrasonore avant une phase d'émission d'énergie d'une sonde d'émission d'énergie afin de mesurer une courbe de signal Doppler de référence et pendant des phases d'arrêt de l'émission d'énergie, entrecoupant les phases d'émission d'énergie pendant une durée comprise entre 1,5 s et 15 s, de préférence entre 1,5 s et 2,5 s, pour mesurer une courbe de signal Doppler reçue après chaque phase d'émission d'énergie.

Le procédé selon l'invention permet alors d'évaluer au cours du traitement par émission d'énergie, et avantageusement en temps réel, les lésions provoquées sur les tissus biologiques par l'émission d'énergie dans la zone de traitement, ainsi que l'évolution du profil de vitesse de l'écoulement de fluide dans un vaisseau aux abords de la zone de traitement. Le traitement par émission d'énergie est alors plus sûr et plus efficace, car le praticien peut évaluer au fur et à mesure l'impact du traitement par émission d'énergie et déterminer lorsqu'il est plus opportun d'arrêter le traitement par émission d'énergie, non seulement en fonction des lésions provoquées aux tissus biologiques mais également en fonction de l'évolution du flux de fluide dans le vaisseau. En effet, dans le cadre de l'invention, l'imagerie ultrasonore, et en particulier l'imagerie Doppler, permet d'avoir une indication au cours du temps, et de préférence en temps réel, de l'impact de l'application d'énergie non seulement sur les tissus biologiques, et en particulier compris dans le champ de visée de la sonde d'imagerie ultrasonore, mais également sur le vaisseau, et en particulier au niveau de la zone de mesure de la sonde d'imagerie ultrasonore, ce qui permet d'évaluer l'état de santé du vaisseau pendant et après l'émission d'énergie, voire d'anticiper une occlusion de celui-ci, et donc de contrôler l'émission d'énergie afin de préserver le vaisseau au sein et aux abords de la zone de traitement. Le procédé selon l'invention est alors plus sûr pour le patient puisqu'il permet notamment d'arrêter le traitement par émission d'énergie avant la sténose du vaisseau, voire avant ou au moment du démarrage du processus de sténose.

Le procédé selon l'invention peut également présenter l'une ou l'autre des caractéristiques suivantes, ou une quelconque combinaison de deux ou plusieurs de ces caractéristiques :
- la zone de mesure de la sonde d'imagerie ultrasonore est positionnée en amont de la zone de traitement,
- la ou les courbe(s) de signal Doppler reçue(s) sont comparée(s) au signal Doppler de référence,
- l'évolution du profil de vitesse est déterminée grâce à la dérivée de la différence entre la(les) courbe(s) de signal Doppler reçue(s) et la(les) courbe(s) de signal Doppler de référence,
- l'arrêt de l'émission de la sonde d'émission d'énergie est pilotée lorsque la courbe de signal Doppler reçue dépasse une valeur seuil,
- la sonde d'émission d'énergie est pilotée en émission selon des phases d'émission d'énergie successives entrecoupées par des phases d'arrêt de l'émission d'énergie, selon un rapport cyclique allant de 30% phase d'émission d'énergie /70% phase d'arrêt d'émission de l'énergie à 90% phase d'émission d'énergie / 10% phase d'arrêt d'émission d'énergie,
- la durée de chaque phase d'émission d'énergie et/ou la durée de chaque phase d'arrêt d'émission d'énergie varie(nt),
- la sonde d'émission d'énergie est une sonde d'émission d'ondes ultrasonores focalisées,
- la zone de traitement est localisée au niveau du cœur, du pancréas, du foie, des reins, ou de la barrière hémato encéphalique, et la sonde d'imagerie ultrasonore est localisée sur un vaisseau sanguin.

### Brève description des dessins

[Fig. 1] La Fig. 1 est un schéma d'un exemple de réalisation d'un dispositif de traitement conforme à l'invention représenté de manière simplifiée en cours de traitement de tissus biologiques.
[Fig. 2] La Fig. 2 est un schéma représentant de manière simplifiée le champ de visée de la sonde d'imagerie ultrasonore, incluant la zone de traitement par émission d'énergie et la zone de mesure de la sonde d'imagerie ultrasonore.
[Fig. 3] La Fig. 3 est un schéma représentant le procédé d'utilisation du dispositif selon l'invention.
[Fig. 4] La Fig. 4 représente un exemple de profil de courbes obtenu (A) avant traitement, (B) à (D) pendant le traitement et (E) en fin de traitement par HIFU de l'artère hépatique d'un modèle porcin.
[Fig. 5] La Fig. 5 représente l'évolution au cours du temps du profil de vitesse de l'écoulement sanguin dans l'artère hépatique d'un modèle porcin (courbe (1) : différence, courbe (2) : dérivée de la différence, droite (S) : valeur seuil définie pour le modèle étudié).

### Description des modes de réalisation

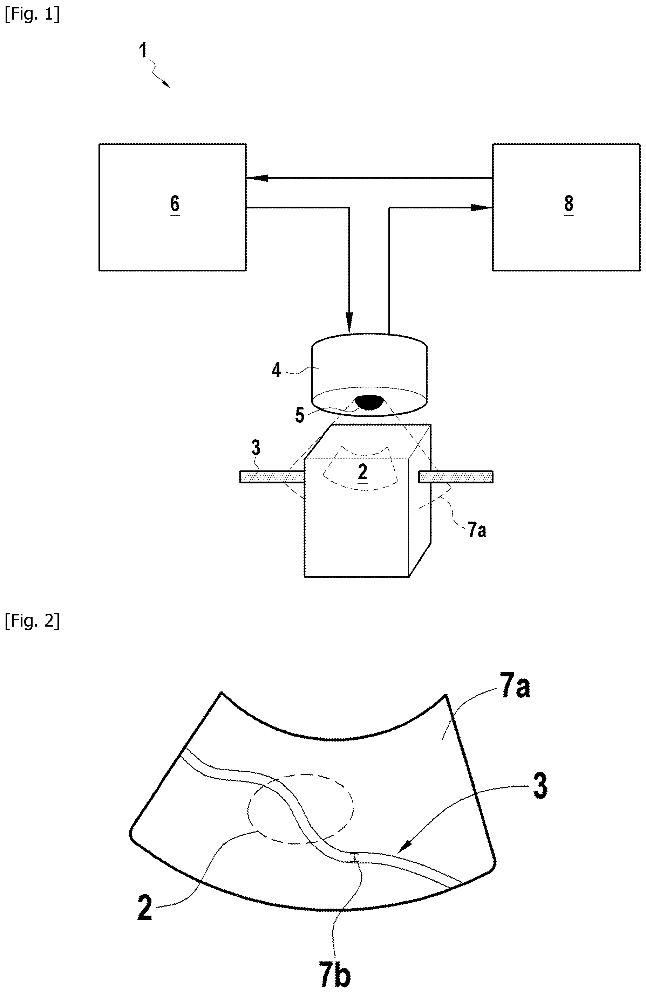

Tel qu'illustré en Fig. 1, l'invention concerne un dispositif **1** pour la caractérisation d'une évolution du profil de vitesse d'écoulement de fluide à la périphérie de la zone de traitement **2** suite à une émission d'énergie.

Dans le contexte de l'invention, « à proximité de », « à la périphérie » et « aux abords » de la zone de traitement **2** sont utilisés de manière interchangeable et signifient localisé à distance de la zone de traitement **2,** c'est-à-dire non inclus dans la zone de traitement **2** mais situé près de la zone de traitement **2,** et notamment à une distance inférieure à 60 mm, typiquement à une distance allant de 5 mm à 60 mm de la zone de traitement **2,** et de préférence de 8 mm à 15 mm.

La zone de traitement **2** comprend des tissus biologiques d'un être vivant. Les tissus biologiques visés sont typiquement des tumeurs, dont la résection par voie chirurgicale pourrait être difficile voire impossible, et qui peuvent être traitées grâce à une émission d'énergie. La zone de traitement **2** comporte au moins un vaisseau **3** pour l'écoulement de fluide, typiquement un vaisseau sanguin, sur lequel des lésions peuvent être provoquées par l'énergie émise par une sonde d'émission d'énergie **4.**

Selon le mode de réalisation illustré à la Fig. 1, le dispositif **1** comporte une sonde d'émission d'énergie **4,** une sonde d'imagerie ultrasonore **5** et un système de commande **6** de la sonde d'émission d'énergie **4** et de la sonde d'imagerie ultrasonore **5.** Bien que préféré, ce mode de réalisation n'est pas limitatif, et il pourrait être envisagé que le dispositif **1** selon l'invention comporte un système de commande configuré pour piloter une sonde d'émission d'énergie **4** et pour piloter une sonde d'imagerie ultrasonore **5,** toutes deux, ou l'une des deux seulement, étant distincte du dispositif **1** selon l'invention et adapté à être raccordée(s) à celui-ci. Dans la description qui suit, le mode de réalisation préféré où le dispositif comporte une sonde d'émission d'énergie **4,** une sonde d'imagerie ultrasonore **5** et un système de commande **6** de la sonde d'émission d'énergie **4** et de la sonde d'imagerie ultrasonore **5** est plus particulièrement détaillé, mais ce qui est explicité ci-dessous s'applique de la même manière à un dispositif ne comportant pas de sonde d'émission d'énergie **4** et/ou de sonde d'imagerie ultrasonore **5,** mais étant destiné à être raccordé à celle(s)-ci, sans sortir du cadre de l'invention.

La sonde d'émission d'énergie **4** peut être de tout type connu en soi pour le traitement de tissus biologiques, tel qu'une sonde d'émission d'électroporation, de radiofréquences, de microondes ou d'ondes ultrasonores focalisées. Selon un mode de réalisation préféré de l'invention, la sonde d'émission d'énergie **4** est une sonde d'émission d'ondes ultrasonores focalisées, ou High Intensity Focused Ultrasound (HIFU) en anglais. Dans ce dernier cas, de manière usuelle, la sonde d'émission d'ondes ultrasonores focalisées comprend un transducteur comportant des émetteurs ultrasonores.

Le système de commande **6** représente l'ensemble des éléments de commande et d'émission. Cela peut être, par exemple, un générateur de signal pour la thérapie et/ou un générateur de signal pour l'imagerie et/ou un ordinateur.

La sonde d'émission d'énergie **4** est pilotée en émission par le système de commande **6.** En d'autres termes, le système de commande **6** provoque l'émission et l'arrêt d'émission d'énergie par la sonde d'émission d'énergie **4.** Ainsi, le système de commande **6** commande des phases d'émission d'énergie **Ai** et des phases d'arrêt de l'émission d'énergie **Bi** alternatives. La durée de ces phases d'émission d'énergie **Ai** et d'arrêt d'émission d'énergie **Bi** successives peut être choisie par l'utilisateur. La durée et l'intensité des phases d'émission de l'énergie **Ai** dépend de la zone de traitement **2,** du tissu biologique cible et des caractéristiques des vaisseaux présents dans et aux abords de la zone de traitement, et ont typiquement une durée allant de 1 s à 120 s. Les phases d'arrêt d'émission d'énergie **Bi** entrecoupant les phases d'émission d'énergie **Ai** peuvent être également choisies par l'utilisateur et ont typiquement une durée allant de 1,5 s à 15 s, de préférence de 1,5 s à 10 s, plus préférentiellement de 1,5 s à 2,5 s. Ces phases d'émission d'énergie **Ai** et d'arrêt d'émission d'énergie **Bi** successives peuvent avoir leurs durées qui varient indépendamment les unes des autres.

Lorsque la sonde d'émission d'énergie **4** est une sonde d'émission d'ondes ultrasonores focalisées, le système de commande **6** comprend un générateur apte à activer ou arrêter le fonctionnement des émetteurs ultrasonores.

Dans le cadre de l'invention, la sonde d'imagerie ultrasonore **5** peut être utilisée selon différents modes. Selon un mode de réalisation particulier, la sonde d'imagerie ultrasonore **5** est une sonde d'imagerie Doppler.

Dans le cadre de l'invention, la sonde d'imagerie ultrasonore **5** est utilisée selon au moins un mode d'imagerie en deux dimensions, de préférence un ou deux mode(s) d'imagerie 2D et notamment deux modes d'imagerie 2D, par exemple en mode Doppler couleur et/ou en mode B. L'utilisation de ce mode permet de visualiser les tissus biologiques dans le champ de visée **7a** de la sonde d'imagerie ultrasonore, qui inclut la zone de traitement **2** tel qu'illustré à la Fig. 1 ou à la Fig. 2. Le choix du ou des modes deux dimensions de la sonde d'imagerie ultrasonore dépend des tissus cibles et du patient traité, et peut être déterminé par l'utilisateur. Ainsi, ce mode 2D permet de visualiser les tissus biologiques, et en particulier la zone de traitement **2** et éventuellement sa périphérie, suite à une émission d'énergie par la sonde d'émission d'énergie **4,** ce qui permet également le ciblage de l'émission d'énergie.

Dans le cadre de l'invention, la sonde d'imagerie ultrasonore **5** est également utilisée selon au moins un mode en une dimension (1D) ou mode courbe Doppler, tel que le mode TM ou le mode Doppler puissance, comme par exemple les sondes Doppler puissance utilisées lors d'un examen cardiaque. Tel qu'illustré sous la forme d'un segment à la Fig. 2, la zone de mesure **7b** de la sonde d'énergie ultrasonore en mode courbe Doppler est placée en dehors de la zone de traitement **2** : la zone de mesure **7b** est positionnée sur un vaisseau traversant la zone de traitement **2,** en amont ou en aval de celle-ci par rapport à l'écoulement de fluide. De préférence, la zone de mesure **7b** de la sonde d'imagerie ultrasonore **5** est placée en amont de la zone de traitement **2** par rapport à l'écoulement du fluide. La sonde d'imagerie ultrasonore en mode courbe Doppler permet ainsi de mesurer le profil de vitesse d'écoulement de fluide dans un vaisseau en amont ou en aval de la zone de traitement **2,** comme par exemple l'écoulement du sang dans un vaisseau sanguin. Par extrapolation, cette mesure donne une indication de l'écoulement de fluide au sein de la zone de traitement **2,** et donc de l'état de santé du vaisseau dans la zone de traitement **2.**

Dans le cadre de l'invention, le champ de visée **7a** de la sonde d'imagerie ultrasonore permet de réaliser un plan d'image dans lequel l'écoulement de fluide est observé selon son axe longitudinal. Dans le cadre de l'invention, l'axe « longitudinal » de l'écoulement de fluide signifie l'axe dans le sens de la longueur du vaisseau où s'écoule le fluide. Il se distingue donc du plan perpendiculaire à l'écoulement de fluide utilisé dans l'art antérieur.

Selon un mode préféré de réalisation, tel qu'illustré à la Fig. 1, la sonde d'émission d'énergie **4** et la sonde d'imagerie ultrasonore **5** sont alignées. Par exemple, la sonde d'émission d'énergie **4** peut être une sonde à ultrasons focalisés intégrant en son centre une sonde d'imagerie ultrasonore **5** de sorte que leur axe acoustique respectif soit confondu. Ce mode de réalisation n'est cependant pas limitatif, et le dispositif peut être tel que ces deux sondes ne sont pas alignées.

Selon le mode de réalisation particulier illustré à la Fig. 1, la sonde d'émission d'énergie **4** est réalisée sous la forme d'un boîtier présentant une face d'émission au centre de laquelle est positionnée la sonde d'imagerie ultrasonore **5.** La sonde d'imagerie ultrasonore **5** et la sonde d'émission d'énergie **4** sont montées solidaires. Tout autre positionnement pourrait néanmoins être envisagé sans sortir du cadre de l'invention.

La sonde d'imagerie ultrasonore **5** est pilotée par le système de commande **6,** en émission et éventuellement en arrêt de l'émission. Plus précisément, le système de commande **6** provoque l'émission de la sonde d'imagerie ultrasonore **5,** et éventuellement l'arrêt de l'émission de la sonde d'imagerie ultrasonore pendant toute la durée ou une partie des phases d'arrêt de l'émission de l'énergie **Bi.**

Selon un premier mode de réalisation, la sonde d'imagerie ultrasonore **5** est pilotée par le système de commande **6** uniquement en émission, c'est-à-dire que le système de commande **6** pilote la sonde d'imagerie ultrasonore **5** en émission continue. Selon un deuxième mode de réalisation, la sonde d'imagerie ultrasonore **5** est pilotée par le système de commande **6,** pendant tout ou partie des phases d'arrêt de l'émission d'énergie **Bi,** en émission ainsi qu'en arrêt de l'émission. Selon ce second mode de réalisation, l'émission de l'énergie ultrasonore n'est pas continue.

Quel que soit le mode de réalisation, pendant les phases d'arrêt de l'émission de l'énergie **Bi,** le signal Doppler reçu est mesuré. Ce signal Doppler mesuré est de préférence enregistré pendant cette même phase d'arrêt d'émission de l'énergie **Bi,** ou peut être enregistré pendant cette même phase d'arrêt d'émission d'énergie et pendant la phase d'émission d'énergie suivante. Le signal Doppler reçu s'entend comme la courbe de signal Doppler obtenue grâce à la sonde d'imagerie ultrasonore utilisée en mode 1D (i.e. mode courbe Doppler) et le plan échographique obtenu grâce à l'imagerie ultrasonore utilisée en mode 2D.

En résumé, le dispositif **1** comporte un système de commande **6** de la sonde d'émission d'énergie **4** et de la sonde d'imagerie ultrasonore **5** qui pilote alternativement la sonde d'émission d'énergie **4** (en émission et en arrêt de l'émission d'énergie) et la sonde d'imagerie ultrasonore **5** (en émission et éventuellement en arrêt de l'émission du signal Doppler, pendant tout ou partie des phases d'arrêt d'émission d'énergie). De préférence, les phases d'émission d'énergie **Ai** et les phases d'arrêt d'émission d'énergie **Bi** successives sont selon un rapport cyclique compris entre 30% phase d'émission d'énergie /70% phase d'arrêt d'émission d'énergie et 90% phase d'émission d'énergie / 10% phase d'arrêt d'émission d'énergie, de préférence 70% phase d'émission d'énergie /30% phase d'arrêt d'émission d'énergie.

La sonde d'imagerie ultrasonore **5** comprend de manière usuelle un transducteur comportant des émetteurs ultrasonores et des récepteurs ultrasonores, ainsi qu'un circuit de traitement **8** du signal Doppler reçu.

Les émetteurs ultrasonores sont avantageusement des émetteurs Doppler pulsé.

Le circuit de traitement **8** représente l'ensemble des éléments de réception et d'analyse. Cela peut être, par exemple, un analyseur de signal pour la thérapie et/ou un analyseur de signal pour l'imagerie et/ou un ordinateur. Le circuit de traitement **8** du signal Doppler reçu permet d'enregistrer les courbes de signaux Doppler reçues. En particulier, une courbe de signal Doppler reçue, nommée courbe de signal Doppler de référence, est mesurée avant une phase d'émission d'énergie **Ai** et enregistré. Cette courbe de signal Doppler de référence peut être la première courbe de signal Doppler reçue mesurée, ou une courbe de signal Doppler reçue ultérieurement. La courbe de signal Doppler de référence peut donc être choisie parmi l'ensemble des courbes de signaux Doppler acquis préalablement à la courbe de signal Doppler reçue considérée. De préférence, la courbe de signal Doppler de référence est la courbe de signal Doppler mesurée avant la première phase d'émission d'énergie **A1.** Dans ce cas, le système de commande **6** pilote dans un premier temps en arrêt la sonde d'émission d'énergie **4,** selon une première phase d'arrêt d'émission d'énergie **B1** au cours de laquelle la courbe de signal Doppler de référence est mesurée par le circuit de traitement **8,** puis pilote en émission la sonde d'émission d'énergie **4** pour une première phase d'émission d'énergie **A1.** Ensuite, des phases d'arrêt et d'émission d'énergie s'enchainent successivement.

Le dispositif **1** selon l'invention permet d'analyser l'évolution de la courbe de signal Doppler reçue, et donc l'évolution du profil de vitesse de l'écoulement de fluide aux abords de la zone de traitement **2.** Pour ce faire, le circuit de traitement **8** de la sonde d'imagerie ultrasonore **5** comporte un système de comparaison de la courbe de signal Doppler reçue à la courbe de signal Doppler de référence. Ainsi, après chaque phase d'émission d'énergie **Ai,** et de préférence au cours de chaque phase d'arrêt d'émission d'énergie **Bi,** la courbe de signal Doppler reçue est enregistrée et comparée à la courbe de signal Doppler de référence. Cette comparaison permet d'identifier des différences entre la courbe de signal Doppler reçue et la courbe de signal Doppler de référence, et ainsi déterminer l'évolution du profil de vitesse de l'écoulement de fluide.

La comparaison entre la courbe de signal Doppler reçue et la courbe de signal Doppler de référence peut être réalisée entre plusieurs courbes de signaux Doppler, et en particulier entre 3 à 5 courbes. De préférence, la comparaison est effectuée entre une moyenne de 3 à 5 courbes de signal Doppler reçues et une moyenne de 3 à 5 courbes de signal Doppler de référence.

La comparaison entre la courbe de signal Doppler reçue et la courbe de signal Doppler de référence peut être réalisée grâce à la différence entre la(les) courbe(s) de signal(aux) Doppler reçue(s) et la(les) courbe(s) de signal(aux) Doppler de référence. La dérivée de la différence entre la(les)courbe(s) de signal(aux) Doppler reçue(s) et la(les) courbe(s) de signal(aux) Doppler de référence peut en outre être calculée. L'étude de la dérivée permet d'identifier plus facilement un changement de comportement d'écoulement de fluide (vitesse, nature du flux, par exemple apparition d'une composante tourbillonnaire du flux).

Selon un mode de réalisation, une valeur seuil peut être enregistrée par le système de traitement **8** afin d'être comparée à la courbe de signal Doppler reçue. Ainsi, lorsque le signal Doppler reçu atteint cette valeur seuil, l'utilisateur pourra en être averti par un système d'avertissement, par exemple par un signal lumineux ou un signal sonore. Il pourrait également être envisagé que le système de commande **6** provoque l'arrêt de l'émission d'énergie lorsque la valeur seuil est atteinte grâce à un système de coupure. Ainsi, lorsque cette valeur seuil représente par exemple une valeur à partir de laquelle le risque de sténose est élevé, le praticien en est averti et peut agir afin d'éviter ou de limiter le risque de sténose, et ainsi préserver au mieux la santé de la personne traitée à l'aide du dispositif **1** selon l'invention.

Cette valeur seuil peut par exemple être une valeur de différence entre la courbe de signal Doppler reçue et la courbe de signal Doppler de référence, ou une valeur de la dérivée de cette différence. Cette valeur seuil dépend des tissus biologiques cibles et du patient, et peut être déterminée par l'utilisateur du dispositif **1,** ou être déterminée grâce à une base de données qui enregistre les courbes de signal Doppler reçues. Dans ce dernier cas, le dispositif **1** est apte à déterminer par lui-même la valeur seuil grâce à la base de données qu'il comporte. Cette base de données peut notamment enregistrer les courbes de signal Doppler reçues pour un même patient lors de différents traitements par exemple, ou bien pour plusieurs patients, pour une même zone de traitement.

L'invention concerne également un procédé de détermination de l'évolution du flux dans un vaisseau en amont ou en aval de la zone de traitement et causée par l'émission préalable d'énergie lors de l'utilisation du dispositif **1** selon l'invention. Dans le cadre de l'invention, l'émission d'énergie provoque une modification du profil de vitesse d'écoulement de fluide au sein et à proximité de la zone de traitement **2,** et le procédé selon l'invention permet, en déterminant l'évolution de ce profil de vitesse au cours du temps, et de préférence en temps réel, d'estimer l'impact sur le vaisseau sanguin au sein et à la périphérie de la zone de traitement de l'émission préalable d'énergie, et plus précisément après chaque émission d'énergie.

Dans le cadre de l'invention, « en temps réel » signifie dans un intervalle de temps faible ou minime par rapport au moment où l'évènement a lieu, et typiquement moins de 15 s, de préférence moins de 10 s, voire moins de 5 s, et idéalement moins de 2 s.

Le dispositif **1** est tel que détaillé plus haut. En particulier, la sonde d'émission d'énergie **4** peut être de tout type connu en soi pour l'émission d'énergie sur des tissus biologiques, telle qu'une sonde d'émission d'électroporation, de radiofréquences, de microondes ou, de préférence, d'ondes ultrasonores focalisées. La sonde d'imagerie ultrasonore **5** est utilisée selon au moins un mode une dimension (i.e. mode courbe Doppler), comme le mode Doppler puissance et/ou le mode TM, et également selon au moins un mode d'imagerie deux dimensions, de préférence un ou deux, comme par exemple le mode Doppler couleur et/ou le mode B. Ainsi, le procédé selon l'invention permet non seulement de visualiser les tissus biologiques, mais également la vitesse d'écoulement de fluide à la périphérie de la zone de traitement **2** par émission d'énergie.

Le procédé selon l'invention comprend une première étape a) au cours de laquelle la zone de mesure **7b** de la sonde d'imagerie ultrasonore **5** (en mode courbe Doppler) est placée en dehors de la zone de traitement **2,** à une distance allant de 5 mm à 60 mm de la zone de traitement **2,** et de préférence de 8 mm à 15 mm, et longitudinalement par rapport à l'axe d'écoulement de fluide dont le profil de vitesse est étudié. La zone de mesure **7b** peut être placée en amont ou en aval de la zone de traitement **2** par rapport à l'écoulement de fluide, de préférence en amont de la zone de traitement **2,** et en particulier à une distance allant de 5 mm à 60 mm, de préférence de 8 mm à 15 mm. Lorsque la zone de mesure **7b** est placée en aval, elle est de préférence placée à une distance allant de 10 mm à 50 mm de la zone de traitement **2.**

De préférence également lors de cette étape a), le champ de visée **7a** de la sonde d'imagerie ultrasonore **5** (en mode d'imagerie 2D) est positionné de sorte à couvrir la zone de traitement **2** et la zone de mesure **7b,** tel qu'illustré à la Fig. 2.

La sonde d'émission d'énergie **4** est pilotée en émission par le système de commande **6,** selon des phases d'émission de l'énergie **Ai** et des phases d'arrêt d'émission de l'énergie **Bi** successives, telles que décrites ci-dessus en lien avec le dispositif **1** selon l'invention.

De manière usuelle pour l'homme du métier, la sonde d'émission d'énergie **4** est positionnée à proximité, au contact ou au sein même de la zone de traitement **2,** selon la nature de la sonde d'émission d'énergie employée.

Selon un mode de réalisation avantageux, et en particulier dans le cas où la sonde d'émission d'énergie est une sonde HIFU, la sonde d'émission d'énergie **4** et la sonde d'imagerie ultrasonore **5** sont positionnées de sorte que le champ de visée **7a** de la sonde d'imagerie ultrasonore **5** et celui de la sonde d'émission d'énergie **4** soient alignés. Cela peut être réalisé par exemple en intégrant la sonde d'imagerie ultrasonore **5** au centre de la sonde d'émission d'énergie **4.** Ce mode de réalisation n'est pas limitatif, et il peut être envisagé de ne pas aligner le champ de visée **7a** de la sonde d'imagerie ultrasonore **5** et celui de la sonde d'émission d'énergie **4** sans sortir du cadre de l'invention.

Le procédé comprend également une étape b) au cours de laquelle la sonde d'imagerie ultrasonore **5** est pilotée en émission, et éventuellement en arrêt de l'émission pendant les phases d'émission d'énergie par le système de commande **6.** Au cours de cette étape la courbe de signal Doppler de référence (avant une phase d'émission d'énergie **Ai)** ainsi que la courbe de signal Doppler reçue (pendant les phases d'arrêt d'émission d'énergie **Bi),** telles que définies précédemment, sont mesurées. Ces signaux Doppler sont mesurés pendant les phases d'arrêt d'émission d'énergie **Bi** et enregistrés pendant lesdites phases d'arrêt d'émission de l'énergie **Bi** et/ou pendant les phases d'émission d'énergie subséquentes.

Avantageusement, les durées des phases d'émission d'énergie **Ai** et des phases d'arrêt d'émission d'énergie **Bi** successives sont selon un rapport allant de 30% phase d'émission d'énergie /70% phase d'arrêt d'émission d'énergie à 90% phase d'émission d'énergie / 10% phase d'arrêt d'émission d'énergie, et de préférence 70% phase d'émission d'énergie /30% phase d'arrêt d'émission d'énergie.

Il peut être envisagé de débuter le procédé selon l'invention par une phase d'émission d'énergie **A1** ou bien de débuter par une phase d'arrêt d'émission d'énergie **B1** au cours de laquelle les signaux Doppler sont émis et reçus. Dans ce dernier cas, la courbe de signal Doppler de référence est de préférence mesurée pendant cette première phase d'arrêt d'émission d'énergie **B1.**

Le nombre de phases d'émission d'énergie **Ai** et d'arrêt d'émission d'énergie **Bi** sont déterminées par l'utilisateur. Plusieurs paramètres peuvent être pris en compte par l'utilisateur pour déterminer le nombre de phases d'émission d'énergie **Ai** et d'arrêt d'émission d'énergie **Bi.** Dans le cas du traitement d'une tumeur, les caractéristiques de la tumeur (type, taille, organe où est localisé la tumeur), du vaisseau (diamètre, vitesse d'écoulement et caractéristiques biologiques) ainsi que le type de sonde d'émission d'énergie utilisée pourront notamment être pris en compte.

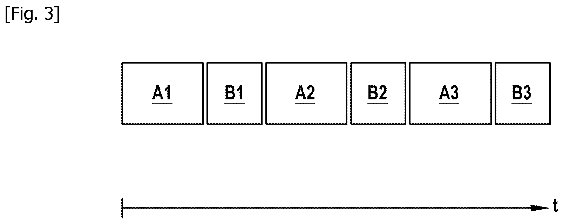

Cette alternance de phases d'émission d'énergie **Ai** et de phases d'arrêt d'émission d'énergie **Bi,** au cours desquelles le signal Doppler est mesuré et enregistré, est illustré à la Fig. 3. Dans le mode de réalisation illustré à la Fig. 3, la sonde d'émission d'énergie **4** est une sonde HIFU, les durées d'émission d'énergie et d'émission du signal Doppler sont identiques à chaque phase et le rapport durée de phase d'émission d'énergie / phase d'arrêt d'émission d'énergie est de 60/40. Selon ce mode de réalisation illustré, trois phases d'émission d'énergie **(A1, A2** et **A3)** sont entrecoupées par trois phases d'arrêt d'émission d'énergie **(B1, B2** et **B3).** Le procédé illustré commence par une phase d'émission d'énergie. Ce mode de réalisation n'est néanmoins pas limitatif, et toute autre sonde d'émission d'énergie, toute autre durée et nombre de phases d'émission d'énergie et d'arrêt d'émission d'énergie pourraient être envisagés sans sortir du cadre de l'invention.

En particulier, il peut être envisagé de faire varier la durée des phases d'émission d'énergie et/ou des phases d'arrêt d'émission d'énergie au cours du temps. Ainsi, selon un mode de réalisation particulier, les phases d'émission d'énergie **Ai** sont de plus en plus courtes au fur et à mesure du traitement, et les phases d'arrêt de l'émission d'énergie **Bi** sont de plus en plus longues. Il peut également être envisagé selon un autre mode de réalisation de faire varier ces durées de manière non linéaire.

Afin de caractériser les modifications de vitesse d'écoulement du fluide dans le vaisseau **3,** la ou les courbe(s) de signal(aux) Doppler reçue(s) est(sont) avantageusement comparé(s) à la courbe de signal Doppler de référence. Comme exposé ci-dessus, chaque courbe de signal Doppler (reçue et/ou de référence) peut être calculée en réalisant une moyenne de plusieurs courbes de signaux Doppler mesurées, et en particulier 3 à 5 courbes de signaux Doppler. Comme détaillé ci-dessus, la comparaison peut être effectuée grâce à la différence entre la ou les courbe(s) de signal(aux) Doppler reçu(es) et de référence, ou grâce à la dérivée de cette différence.

La mesure du signal Doppler reçu est effectuée, à intervalle de temps régulier ou non, à chaque phase d'arrêt de l'émission d'énergie **Bi.** Ainsi, la mesure de la courbe de signal Doppler reçue et sa comparaison à la courbe du signal Doppler de référence permettent de suivre l'évolution de l'écoulement de fluide au fur et à mesure, et avantageusement en temps réel. Cela permet alors au praticien de suivre l'évolution de l'impact de l'émission d'énergie sur le vaisseau étudié présent dans la zone de traitement **2.**

Dans l'objectif de suivre encore plus aisément et de manière encore plus sûre pour le patient l'évolution du profil de vitesse de l'écoulement de fluide provoquée par l'émission d'énergie, il est possible que le circuit de traitement **8** comporte un système de comparaison de la courbe du signal Doppler reçue à une valeur seuil, tel que décrit précédemment. Ainsi, dès que cette valeur seuil est atteinte, la sonde d'émission d'énergie **4** peut être d'office arrêtée par un système de coupure.

Le procédé selon l'invention permet avantageusement de traiter une grande variété de tissus biologiques parmi lesquels peuvent être cités le cœur, le pancréas, le foie, les reins, ou la barrière hémato encéphalique. Dans ce cas, la sonde d'imagerie ultrasonore permet avantageusement de mesurer le signal Doppler d'un vaisseau sanguin.

Le dispositif selon l'invention permet avantageusement de réaliser des thérapies ablatives, et en particulier l'ablation de tumeurs, de manière non invasive et plus sûre pour le patient puisque le risque de sténose est limité voir supprimé.

### Exemples

Le dispositif selon l'invention a été utilisé pour traiter le pancréas d'un modèle porcin. La zone de mesure est située sur l'artère hépatique, en amont de la zone de traitement.

L'émission d'énergie est réalisée grâce à une sonde HIFU.

La sonde HIFU est pilotée en émission selon des phases d'émission d'énergie de 10 s chacune, pendant 36 phases d'émission d'énergie (pour un total d'émission HIFU de 360 s).

La séquence débute par une phase d'émission d'énergie, suivie d'une phase d'arrêt d'émission d'énergie. La sonde HIFU est pilotée en arrêt de l'émission d'énergie **(Bi)** pendant les phases d'imagerie ultrasonore d'une durée de 15 secondes, et en émission d'énergie pendant les phases d'émission d'énergie **(Ai).** La sonde d'imagerie ultrasonore est pilotée en arrêt d'acquisition du signal pendant les phases d'émission d'énergie **(Ai)** et en acquisition du signal pendant une partie (2 secondes) des phases d'arrêt d'émission d'énergie **(Bi).**

La sonde d'imagerie ultrasonore est une sonde d'imagerie Doppler (1D et 2D) utilisée en mode Doppler puissance et en mode combiné mode B/Doppler couleur.

La sonde d'imagerie Doppler et la sonde HIFU sont alignées.

L'algorithme analyse les courbes de signal Doppler 1D. La courbe temporelle et son évolution au cours du traitement sont interprétées par l'algorithme de calcul. L'intégralité du signal est exploitée.

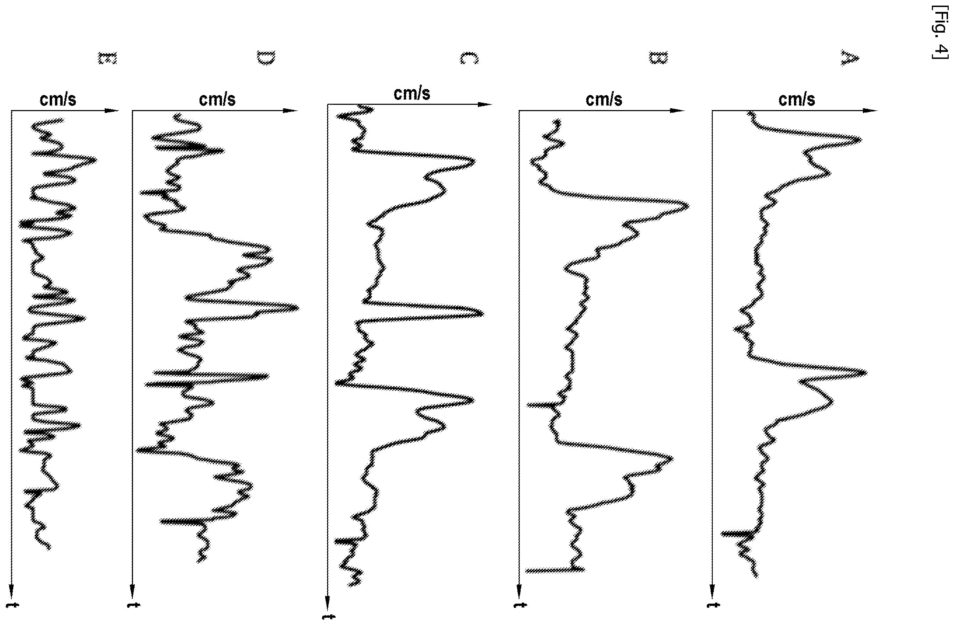

La Fig. 4 représente l'évolution des changements du signal au cours du temps par rapport à une courbe de signal Doppler de référence (signal A). Les signaux A à E correspondent à des courbes de signal Doppler acquis au début (signal A), au cours (signaux B à D) ou en fin de traitement (signal E).

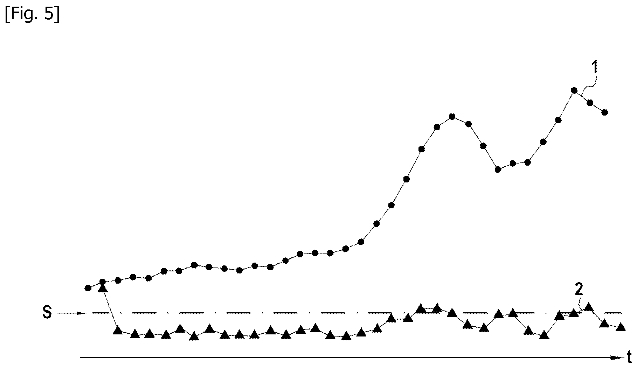

Une courbe représentant l'évolution de la différence du profil de vitesse au cours du traitement selon la Fig. 4 est obtenue en réalisant une moyenne de cinq valeurs de différence (Fig. 5). La courbe (1) représente l'évolution de la différence entre la courbe de signal Doppler reçue et la courbe de signal Doppler de référence, sur une moyenne de 5 mesures. La courbe (2) trace la pente de la courbe de différence (1). Elle correspond donc à la dérivée de la différence. La ligne **S** détermine la valeur seuil censée définir la limite que la pente ne doit pas dépasser pour assurer un traitement évitant la perte du signal artériel. Dans l'exemple illustré, la valeur seuil est fixée à 1.

Plus les signaux sont décorrélés, plus la différence est grande. La décorrélation progressive des signaux au cours du temps se traduit par une augmentation croissante de la courbe de différence (1). Si la différence est trop importante et augmente rapidement, alors la pente (2) tend à dépasser ce seuil car les signaux deviennent extrêmement perturbés par rapport aux signaux de référence.

## Revendications

1. Dispositif **(1)** pour la caractérisation d'une évolution du profil de vitesse d'écoulement de fluide à la périphérie d'une zone de traitement **(2),** suite à l'émission d'énergie par une sonde d'émission d'énergie **(4),** grâce à une sonde d'imagerie ultrasonore **(5)** dont la zone de mesure **(7b)** est placée en dehors de la zone de traitement **(2),** ladite sonde d'imagerie ultrasonore **(5)** comprenant un circuit de traitement **(8)** du signal Doppler reçu, la sonde d'imagerie ultrasonore **(5)** étant positionnée pour réaliser un plan d'image dans lequel l'écoulement de fluide est observé selon son axe longitudinal, ledit dispositif **(1)** comportant un système de commande **(6)** de la sonde d'émission d'énergie **(4)** et de la sonde d'imagerie ultrasonore **(5),** configuré pour :
- commander en émission la sonde d'émission d'énergie **(4)** selon des phases d'émission d'énergie **(Ai)** successives entrecoupées par des phases d'arrêt de l'émission d'énergie **(Bi),** lesdites phases d'arrêt de l'émission d'énergie **(Bi)** ayant une durée allant de 1,5 s à 15 s et de préférence de 1,5 s à 2,5 s,
- piloter en émission la sonde d'imagerie ultrasonore **(5)** de telle sorte que sa zone de mesure **(7b)** est placée en dehors de la zone de traitement, avant une phase d'émission d'énergie **(Ai)** afin de mesurer un signal Doppler de référence et pendant les phases d'arrêt de l'émission d'énergie **(Bi)** pour mesurer un signal Doppler reçu après chaque phase d'émission d'énergie **(Ai),**
- et permettre de fournir un suivi de l'évolution du profil de vitesse d'écoulement du fluide issu de l'imagerie ultrasonore.

2. Dispositif **(1)** selon la revendication 1 selon lequel la sonde d'imagerie ultrasonore **(5)** donne accès à au moins un mode courbe Doppler, tel que le mode Doppler 1D ou le mode TM, pour mesurer une évolution du profil de vitesse d'écoulement de fluide au niveau de la zone de mesure **(7b),** et au moins un mode d'imagerie en deux dimensions, tel que le mode B ou le mode Doppler couleur, pour visualiser des tissus biologiques de la zone de traitement **(2).**

3. Dispositif selon la revendication 1 ou 2 selon lequel le système de commande **(6)** pilote en émission la sonde d'émission d'énergie **(4)** selon des phases d'émission d'énergie **(Ai)** successives entrecoupées par des phases d'arrêt de l'émission d'énergie **(Bi),** selon un rapport cyclique compris entre 30% phase d'émission d'énergie /70% phase d'arrêt d'émission d'énergie et 90% phase d'émission d'énergie / 10% phase d'arrêt d'émission d'énergie.

4. Dispositif **(1)** selon l'une des revendications 1 à 3 selon lequel le circuit de traitement **(8)** du signal Doppler analyse l'évolution du profil de vitesse obtenu par effet Doppler.

5. Dispositif **(1)** selon l'une des revendications 2 à 4 selon lequel le circuit de traitement **(8)** du signal Doppler comporte un système de comparaison de la courbe du signal Doppler reçue et la courbe du signal Doppler de référence après chaque phase d'émission d'énergie **(Ai),** pour déterminer l'évolution du profil de vitesse obtenu par l'imagerie ultrasonore.

6. Dispositif **(1)** selon la revendication 5 selon lequel le système de comparaison du circuit de traitement **(8)** du signal Doppler réalise la comparaison entre une moyenne de 3 à 5 courbes de signaux Doppler reçues et une moyenne de 3 à 5 courbes de signaux Doppler de référence.

7. Dispositif **(1)** selon la revendication 5 ou 6 selon lequel le circuit de traitement **(8)** du signal Doppler détermine l'évolution du profil de vitesse grâce à la différence entre la(les) courbe(s) de signal(aux) Doppler reçue(s) et la(les) courbe(s) de signal(aux) Doppler de référence.

8. Dispositif **(1)** selon la revendication 5 ou 6 selon lequel le circuit de traitement **(8)** du signal Doppler détermine l'évolution du profil de vitesse grâce à la dérivée de la différence entre la(les) courbe(s) de signal(aux) Doppler reçue(s) et la(les) courbe(s) de signal(aux) Doppler de référence.

9. Dispositif **(1)** selon l'une des revendications 2 à 8 selon lequel le circuit de traitement du signal Doppler comporte un système de comparaison à une valeur seuil de la (des) courbe(s) du signal Doppler reçue(s), de la différence entre la(les) courbe(s) de signal(aux) Doppler reçue(s) et la(les) courbe(s) de signal(aux) Doppler de référence, ou de la dérivée de la différence entre la(les) courbe(s) de signal(aux) Doppler reçue(s) et la(les) courbe(s) de signal(aux) Doppler de référence.

10. Dispositif selon la revendication 9 selon lequel le circuit traitement **(8)** comporte un système de coupure arrêtant la sonde d'émission d'énergie **(4)** lorsque la(les) courbe(s) de signal Doppler reçue(s), la valeur de la différence entre la(les) courbe(s) de signal(aux) Doppler reçue(s) et la(les) courbe(s) de signal(aux) Doppler de référence, ou la valeur de la dérivée de la différence entre la(les) courbe(s) de signal(aux) Doppler reçue(s) et la(les) courbe(s) de signal(aux) Doppler de référence atteint ladite valeur seuil.

11. Dispositif **(1)** selon la revendication 9 ou 10 comportant une base de données enregistrant les courbes de signal Doppler reçues, la différence des entre la(les) courbe(s) de signal(aux) Doppler reçu(s) et la(les) courbe(s) de signal(aux) Doppler de référence, et/ou la valeur de la dérivée de la différence entre la(les) courbe(s) de signal(aux) Doppler reçue(s) et la(les) courbe(s) de signal(aux) Doppler de référence afin de déterminer par lui-même la valeur seuil.

12. Dispositif **(1)** selon l'une quelconque des revendications précédentes selon lequel la sonde d'émission d'énergie **(4)** est une sonde d'émission d'ondes ultrasonores focalisées.

13. Dispositif **(1)** selon l'une quelconque des revendications 1 à 11 selon lequel la sonde d'émission d'énergie **(4)** est une sonde d'émission d'électroporation, de radiofréquences ou une sonde d'émission de microondes.

14. Procédé de caractérisation d'une évolution du profil de vitesse d'écoulement de fluide à la périphérie d'une zone de traitement suite à l'émission d'énergie à l'aide du dispositif **(1)** selon l'une quelconque des revendications précédentes, comprenant les étapes suivantes :
a- positionnement de la zone de mesure **(7b)** de la sonde d'imagerie ultrasonore **(5)** en dehors de la zone de traitement **(2),** à 5 à 60 mm de la zone de traitement **(2),** et longitudinalement par rapport à l'axe d'écoulement du fluide,
b- pilotage en émission la sonde d'imagerie ultrasonore **(5)** avant une phase d'émission d'énergie **(Ai)** d'une sonde d'émission d'énergie **(4)** afin de mesurer une courbe de signal Doppler de référence et pendant des phases d'arrêt de l'émission d'énergie **(Bi),** entrecoupant les phases d'émission d'énergie **(Ai)** pendant une durée comprise entre 1,5 s et 15 s, de préférence entre 1,5 s et 2,5 s, pour mesurer une courbe de signal Doppler reçue après chaque phase d'émission d'énergie **(Ai).**

15. Procédé selon la revendication précédente selon lequel la zone de mesure **(7b)** de la sonde d'imagerie ultrasonore **(5)** est positionnée en amont de la zone de traitement **(2).**

16. Procédé selon l'une quelconque des revendications 14 à 15 selon lequel la ou les courbe(s) de signal Doppler reçue(s) sont comparée(s) au signal Doppler de référence.

17. Procédé selon la revendication précédente selon lequel l'évolution du profil de vitesse est déterminée grâce à la dérivée de la différence entre la(les) courbe(s) de signal Doppler reçue(s) et la(les) courbe(s) de signal Doppler de référence.

18. Procédé selon l'une quelconque des revendications 14 à 17 selon lequel la durée de chaque phase d'émission d'énergie **(Ai)** et/ou la durée de chaque phase d'arrêt d'émission d'énergie **(Bi)** varie(nt).

19. Procédé selon l'une des revendications 14 à 18 selon lequel la sonde d'émission d'énergie **(4)** est une sonde d'émission d'ondes ultrasonores focalisées.

20. Procédé selon l'une quelconque des revendications 14 à 19 selon lequel la zone de traitement **(2)** est localisée au niveau du cœur, du pancréas, du foie, des reins, ou de la barrière hémato encéphalique, et la sonde d'imagerie ultrasonore **(5)** est localisée sur un vaisseau sanguin.

## Patentansprüche

1. Vorrichtung (1) für die Charakterisierung einer Entwicklung des Geschwindigkeitsprofils einer Fluidströmung in der Peripherie einer Behandlungszone (2), nach der Emission von Energie durch eine Energieemissionssonde (4), mittels einer Ultraschall-Bildgebungssonde (5), deren Messzone (7b) außerhalb der Behandlungszone (2) platziert ist, wobei die Ultraschall-Bildgebungssonde (5) eine Schaltung zur Verarbeitung (8) des empfangenen Doppler-Signals umfasst, wobei die Ultraschall-Bildgebungssonde (5) positioniert wird, um eine Bildebene zu verwirklichen, in welcher die Fluidströmung entlang ihrer Längsachse beobachtet wird, wobei die Vorrichtung (1) ein Steuersystem (6) der Energieemissionssonde (4) und der Ultraschall-Bildgebungssonde (5) beinhaltet, das dazu ausgestaltet ist:
- die Energieemissionssonde (4) im Emissionsbetrieb gemäß aufeinanderfolgenden Energieemissionsphasen (Ai) zu steuern, die von Phasen des Stopps der Energieemission (Bi) unterbrochen werden, wobei die Phasen des Stopps der Energieemission (Bi) eine Dauer aufweisen, die von 1,5 s bis 15 s und vorzugsweise von 1,5 s bis 2,5 s reichen,
- Ansteuern der Ultraschall-Bildgebungssonde (5) im Emissionsbetrieb auf solche Weise, dass ihre Messzone (7b) außerhalb der Behandlungszone platziert ist, vor einer Energieemissionsphase (Ai), um ein Referenz-Doppler-Signal zu messen, und während der Phasen des Stopps der Energieemission (Bi) nach jeder Energieemissionsphase (Ai), um ein empfangenes Doppler-Signal zu messen,
- und Erlauben, eine Nachverfolgung der Entwicklung des Geschwindigkeitsprofils der Fluidströmung aus der Ultraschallbildgebung zu liefern.

2. Vorrichtung (1) nach Anspruch 1, wobei die Ultraschall-Bildgebungssonde (5) Zugriff auf zumindest einen Doppler-Kurvenmodus, wie den 1D-Dopplermodus oder den TM-Modus, zum Messen einer Entwicklung des Geschwindigkeitsprofils der Fluidströmung an der Messzone (7b), und zumindest einen zweidimensionalen Bildgebungsmodus, wie den B-Modus oder den Farb-Dopplermodus, zum Visualisieren von biologischem Gewebe der Behandlungszone (2), gewährt.

3. Vorrichtung nach Anspruch 1 oder 2, wobei das Steuersystem (6) die Energieemissionssonde (4) im Emissionsbetrieb gemäß aufeinanderfolgenden Energieemissionsphasen (Ai), die von Phasen des Stopps der Energieemission (Bi) unterbrochen werden, gemäß einem zyklischen Verhältnis in dem Bereich zwischen 30 % Energieemissionsphase /70 % Phase des Stopps der Energieemission und 90 % Energieemissionsphase / 10 % Phase des Stopps der Energieemission steuert.

4. Vorrichtung (1) nach einem der Ansprüche 1 bis 3, wobei die Schaltung zur Verarbeitung (8) des Doppler-Signals die Entwicklung des Geschwindigkeitsprofils analysiert, das durch den Doppler-Effekt erhalten wird.

5. Vorrichtung (1) nach einem der Ansprüche 2 bis 4, wobei die Schaltung zur Verarbeitung (8) des Doppler-Signals ein System zum Vergleich der empfangenen Kurve des Doppler-Signals und der Kurve des Referenz-Doppler-Signals nach jeder Energieemissionsphase (Ai) beinhaltet, um die Entwicklung des Geschwindigkeitsprofils zu bestimmen, das durch die Ultraschall-Bildgebung erhalten wird.

6. Vorrichtung (1) nach Anspruch 5, wobei das Vergleichssystem der Schaltung zur Verarbeitung (8) des Doppler-Signals den Vergleich zwischen einem Durchschnitt von 3 bis 5 empfangenen Kurven von Doppler-Signalen und einem Durchschnitt von 3 bis 5 Kurven von Referenz-Doppler-Signalen durchführt.

7. Vorrichtung (1) nach Anspruch 5 oder 6, wobei die Schaltung zur Verarbeitung (8) des Doppler-Signals die Entwicklung des Geschwindigkeitsprofils mittels der Differenz zwischen der/den empfangenen Kurve/n des/der Doppler-Signals/-e und der/den Kurve/n des/der Referenz-Doppler-Signals/-e bestimmt.

8. Vorrichtung (1) nach Anspruch 5 oder 6, wobei die Schaltung zur Verarbeitung (8) des Doppler-Signals die Entwicklung des Geschwindigkeitsprofils mittels der Ableitung der Differenz zwischen der/den empfangenen Kurve/n des/der Doppler-Signals/-e und der/den Kurve/n des/der Referenz-Doppler-Signals/-e bestimmt.

9. Vorrichtung (1) nach einem der Ansprüche 2 bis 8, wobei die Schaltung zur Verarbeitung des Doppler-Signals ein System zum Vergleich der empfangenen Kurve/n des/der Doppler-Signals/-e, der Differenz zwischen der/den empfangenen Kurve/n des/der Doppler-Signals/-e und der/den Kurve/n des/der Referenz-Doppler-Signals/-e, oder der Ableitung der Differenz zwischen der/den empfangenen Kurve/n des/der Doppler-Signals/-e und der/den Kurve/n des/der Referenz-Doppler-Signals/-e, mit einem Schwellenwert beinhaltet.

10. Vorrichtung nach Anspruch 9, wobei die Schaltung zur Verarbeitung (8) ein Abschaltsystem beinhaltet, das die Energieemissionssonde (4) stoppt, wenn die empfangenen Kurve/n des/der Doppler-Signals/-e, der Wert der Differenz zwischen der/den empfangenen Kurve/n des/der Doppler-Signals/-e und der/den Kurve/n des/der Referenz-Doppler-Signals/-e, oder der Wert der Ableitung der Differenz zwischen der/den empfangenen Kurve/n des/der Doppler-Signals/-e und der/den Kurve/n des/der Referenz-Doppler-Signals/-e den Schwellenwert erreicht/erreichen.

11. Vorrichtung (1) nach Anspruch 9 oder 10, die eine Datenbank beinhaltet, welche die empfangenen Kurven des Doppler-Signals, die Differenz zwischen der/den empfangenen Kurve/n des/der Doppler-Signals/-e und der/den Kurve/n des/der Referenz-Doppler-Signals/-e, oder den Wert der Ableitung der Differenz zwischen der/den empfangenen Kurve/n des/der Doppler-Signals/-e und der/den Kurve/n des/der Referenz-Doppler-Signals/-e speichert, um dadurch den Schwellenwert zu bestimmen.

12. Vorrichtung (1) nach einem der vorhergehenden Ansprüche, wobei die Energieemissionssonde (4) eine Sonde zur Emission von fokussierten Ultraschallwellen ist.

13. Vorrichtung (1) nach einem der Ansprüche 1 bis 11, wobei die Energieemissionssonde (4) eine Elektroporations-, Funkfrequenz- oder eine Mikrowellen-Emissionssonde ist.

14. Verfahren zur Charakterisierung einer Entwicklung des Geschwindigkeitsprofils einer Fluidströmung in der Peripherie einer Behandlungszone nach der Energieemission mittels der Vorrichtung (1) nach einem der vorhergehenden Ansprüche, umfassend die folgenden Schritte:
a- Positionierung der Messzone (7b) der Ultraschall-Bildgebungssonde (5) außerhalb der Behandlungszone (2), 5 bis 60 mm von der Behandlungszone (2) entfernt und in Längsrichtung in Bezug auf die Strömungsachse des Fluids,
b- Ansteuerung der Ultraschall-Bildgebungssonde (5) im Emissionsbetrieb vor einer Energieemissionsphase (Ai) einer Energieemissionssonde (4), um eine Referenz-Doppler-Signalkurve zu messen, und während der Phasen des Stopps der Energieemission (Bi), welche die Energieemissionsphasen (Ai) während einer Dauer zwischen 1,5 s und 15 s, vorzugsweise zwischen 1,5 s und 2,5 s, unterbrechen, um eine Doppler-Signalkurve zu messen, die nach jeder Energieemissionsphase (Ai) empfangen wird.

15. Verfahren nach dem vorhergehenden Anspruch, wobei die Messzone (7b) der Ultraschall-Bildgebungssonde (5) der Behandlungszone (2) vorgelagert positioniert wird.

16. Verfahren nach einem der Ansprüche 14 bis 15, wobei die empfangene/n Doppler-Signalkurve/n mit dem Referenz-Doppler-Signal verglichen wird/werden.

17. Verfahren nach dem vorhergehenden Anspruch, wobei die Entwicklung des Geschwindigkeitsprofils mittels der Ableitung der Differenz zwischen den empfangene/n Doppler-Signalkurve/n und der/den Referenz-Doppler-Signalkurven bestimmt wird.

18. Verfahren nach einem der Ansprüche 14 bis 17, wobei die Dauer jeder Energieemissionsphase (Ai) und/oder die Dauer jeder Phase des Stopps der Energieemission (Bi) variiert/-en.

19. Verfahren nach einem der Ansprüche 14 bis 18, wobei die Energieemissionssonde (4) eine Sonde zur Emission von fokussierten Ultraschallwellen ist.

20. Verfahren nach einem der Ansprüche 14 bis 19, wobei die Behandlungszone (2) am Herzen, der Pankreas, der Leber, an den Nieren oder der Blut-Hirn-Schranke lokalisiert ist und die Ultraschall-Bildgebungssonde (5) auf einem Blutgefäß lokalisiert ist.

## Claims

1. A device **1.** for characterizing an evolution in the fluid flow rate profile at the periphery of a treatment area **(2),** following the emission of energy by an energy emission probe **(4),** thanks to an ultrasound imaging probe **(5)** whose measurement area **(7b)** is placed outside the treatment area **(2),** said ultrasound imaging probe **(5)** comprising a circuit **(8)** for processing the received Doppler signal, the ultrasound imaging probe **(5)** being positioned to produce an image plane in which the flow of fluid is observed along its longitudinal axis, said device **(1)** including a system **(6)** for controlling the energy emission probe **(4)** and the ultrasound imaging probe **(5),** configured to:
- control the energy emission probe **(4)** in emission according to successive energy emission phases **(Ai)** interspersed with energy emission stop phases **(Bi),** said energy emission stop phases **(Bi)** having a duration ranging from 1.5 s to 15 s and preferably from 1.5 s to 2.5 s,
- drive the ultrasound imaging probe **(5)** in emission such that its measurement area (7b) is placed outside the treatment area before an energy emission phase **(Ai)** in order to measure a reference Doppler signal and during the energy emission stop phases **(Bi)** to measure a received Doppler signal after each energy emission phase **(Ai),**
- and allow to provide monitoring of the evolution of the fluid flow rate profile resulting from the ultrasound imaging.

2. The device **1.** according to claim 1, wherein the ultrasound imaging probe **(5)** gives access to at least one Doppler curve mode, such as the 1D Doppler mode or the TM mode, to measure an evolution in the fluid flow rate profile at the measurement area **(7b),** and at least one two-dimensional imaging mode, such as the B mode or the color Doppler mode, to visualize biological tissues of the treatment area **(2).**

3. The device according to claim 1 or 2, wherein the control system **6.** controls the energy emission probe **(4)** in emission according to successive energy emission phases **(Ai)** interspersed with energy emission stop phases **(Bi),** according to a duty cycle comprised between 30% energy emission phase /70% energy emission stop phase and 90% energy emission phase/10% energy emission stop phase.

4. The device **1.** according to one of claims 1 to 3, wherein the Doppler signal processing circuit **(8)** analyzes the evolution of the rate profile obtained by Doppler effect.

5. The device **1.** according to one of claims 2 to 4, wherein the Doppler signal processing circuit **(8)** includes a system for comparing the received Doppler signal curve and the reference Doppler signal curve after each energy emission phase **(Ai),** to determine the evolution of the rate profile obtained by the ultrasound imaging.

6. The device **1.** according to claim 5 according to which the comparison system of the Doppler signal processing circuit **(8)** carries out the comparison between an average of 3 to 5 received Doppler signal curves and an average of 3 to 5 reference Doppler signal curves.

7. The device **1.** according to claim 5 or 6, according to which the Doppler signal processing circuit **(8)** determines the evolution of the rate profile thanks to the difference between the received Doppler signal(s) curve(s) and the reference Doppler signal(s) curve(s).

8. The device **1.** according to claim 5 or 6, according to which the Doppler signal processing circuit **(8)** determines the evolution of the rate profile thanks to the derivative of the difference between the received Doppler signal(s) curve(s) and the reference Doppler signal(s) curve(s).

9. The device **1.** according to one of claims 2 to 8, wherein the Doppler signal processing circuit includes a system for comparing with a threshold value the received Doppler signal curve(s), the difference between the received Doppler signal(s) curve(s) and the reference Doppler signal(s) curve(s), or the derivative of the difference between the received Doppler signal(s) curve(s) and the reference Doppler signal(s) curve(s).

10. The device according to claim 9, according to which the processing circuit **(8)** includes a cut-off system stopping the energy emission probe **(4)** when the received Doppler signal(s) curve(s), the value of the difference between the received Doppler signal(s) curve(s) and the reference Doppler signal(s) curve(s), or the value of the derivative of the difference between the received Doppler signal(s) curve(s) and the reference Doppler signal(s) curve(s) reaches said threshold value.

11. The device **1.** according to claim 9 or 10 including a database recording the received Doppler signal curves, the difference between the received Doppler signal(s) curve(s) and the reference Doppler signal(s) curve(s), and/or the value of the derivative of the difference between the received Doppler signal(s) curve(s) and the reference Doppler signal(s) curve(s) in order to determine the threshold value by itself.

12. The device **1.** according to any one of the preceding claims, wherein the energy emission probe **(4)** is a focused ultrasound wave emission probe.

13. The device **1.** according to any one of claims 1 to 11 wherein the energy emission probe **(4)** is an electroporation emission probe, radio frequency emission probe or a microwave emission probe.

14. A method for characterizing an evolution of the fluid flow rate profile at the periphery of a treatment area following the emission of energy using the device (1) according to any one of the preceding claims, comprising the following steps:
a- positioning the measurement area **(7b)** of the ultrasound imaging probe **(5)** outside the treatment area **(2),** 5 to 60 mm from the treatment area **(2),** and longitudinally relative to the fluid flow axis,
b- driving the ultrasound imaging probe **(5)** in emission before an energy emission phase **(Ai)** of an energy emission probe **(4)** in order to measure a reference Doppler signal curve and during energy emission stop phases **(Bi),** interspersing the energy emission phases **(Ai)** for a duration comprised between 1.5 s and 15 s, preferably between 1.5 s and 2.5 s, to measure a
received Doppler signal curve after each energy emission phase **(Ai).**

15. The method according to the preceding claim, wherein the measurement area **(7b)** of the ultrasound imaging probe **(5)** is positioned upstream of the treatment area **(2).**

16. The method according to any one of claims 14 to 15 wherein the received Doppler signal curve(s) is/are compared with the reference Doppler signal.

17. The method according to the preceding claim, wherein the evolution of the rate profile is determined thanks to the derivative of the difference between the received Doppler signal curve(s) and the reference Doppler signal curve(s).

18. The method according to any one of claims 14 to 17, according to which the duration of each energy emission phase **(Ai)** and/or the duration of each energy emission stop phase **(Bi)** varies/vary.

19. The method according to one of claims 14 to 18, wherein the energy emission probe **(4)** is a focused ultrasound wave emission probe.

20. The method according to any one of claims 14 to 19, wherein the treatment area **(2)** is located at the heart, the pancreas, the liver, the kidneys, or the blood-brain barrier, and the ultrasound imaging probe **(5)** is located on a blood vessel.
